(19) Europäisches Patentamt
European Patent Office
Office européen des brevets

(11) EP 2 500 016 A1

# (12) EUROPEAN PATENT APPLICATION

(43) Date of publication:
**19.09.2012 Bulletin 2012/38**

(21) Application number: **11382072.4**

(22) Date of filing: **18.03.2011**

(51) Int Cl.:
***A61K 31/135*** (2006.01) ***A61K 31/4402*** (2006.01)
***A61K 9/00*** (2006.01) ***A61K 9/08*** (2006.01)
***A61K 9/10*** (2006.01) ***A61K 9/20*** (2006.01)
***A61K 9/48*** (2006.01) ***A61K 47/48*** (2006.01)
*A61P 25/20* (2006.01)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Laboratorios del. Dr. Esteve, S.A.**
**08041 Barcelona (ES)**

(72) Inventors:
- **Font Cot, Jordi**
  **08820 El Prat de Llobregat (Barcelona) (ES)**
- **Falivene Aldea, Albert**
  **08010 Barcelona (ES)**
- **Soler Ranzani, Luis**
  **08013 Barcelona (ES)**
- **Casadevall Pujals, Gemma**
  **08019 Barcelona (ES)**

(74) Representative: **Carpintero Lopez, Francisco et al**
**Herrero & Asociados, S.L.**
**Alcalá 35**
**28014 Madrid (ES)**

## (54) Doxylamine resinate complex

(57) The invention refers to a new doxylamine resinate complex comprising doxylamine bound to a weakly acidic synthetic cation exchange resin, such as a copolymer of methacrylic acid and divinylbenzene The invention discloses a process for its preparation which comprises contacting a weakly acidic synthetic cation exchange resin with doxylamine or a pharmaceutically acceptable salt thereof and its use for the preparation of pharmaceutical compositions, such as chewable tablets or oral disintegrating tablets of immediate release. Further the invention refers to the doxylamine resinate complex for use as a medicament, preferably as a medicament for use in the treatment of occasional insomnia

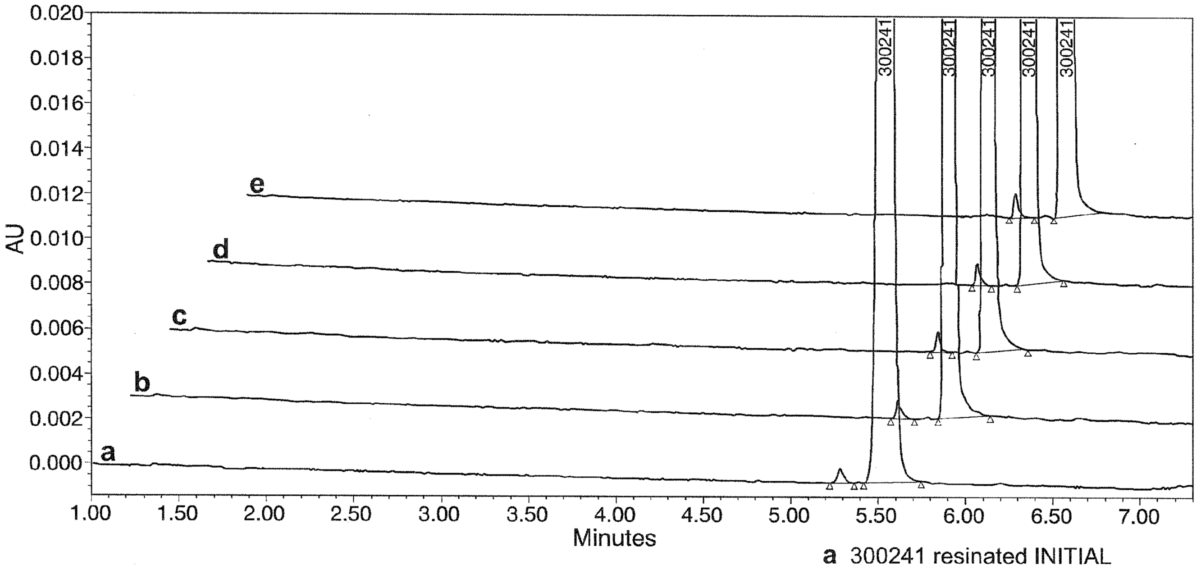


**a** 300241 resinated INITIAL
**b** Resinated 15d 40°C/75% RH
**c** Resinated 15d 50°C
**d** Resinated 30d 40°C/75% RH
**e** Resinated 30d 50°C

**FIG. 2**



Printed by Jouve, 75001 PARIS (FR)

## Description

### FIELD OF INVENTION

The present invention relates to a doxylamine resinate complex formed between doxylamine and a synthetic weakly acidic cation exchange resin, a process for preparation of the same and its use as a medicament for the treatment of occasional insomnia. The invention also relates to pharmaceutical formulations comprising said doxylamine resinate complex, such as oral pharmaceutical formulations.

### BACKGROUND OF THE INVENTION

Doxylamine is an ethanolamine-based antihistamine used in the management of insomnia and, in combination with antitussives and decongestants, in the relief of cough and cold symptoms. It is also used in combination with the analgesics paracetamol (acetaminophen) and codeine as an analgesic/calmative preparation, and is prescribed in combination with vitamin $B_6$ (pyridoxine) to prevent morning sickness in pregnant women.

Doxylamine, whose chemical name is (*RS*)-*N,N*-dimethyl-2-(1-phenyl-1-(pyridin-2-yl)ethoxy)ethanamine, has the following formula:

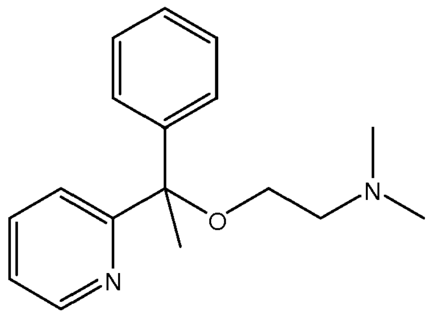

Doxylamine

Doxylamine is available in the market as a physiologically acceptable salt, particularly as a succinate.

One problem of doxylamine succinate is its high hygroscopicity (deliquescent) which can result in a need for special manufacturing conditions or a difficulty to keep the properties of the dosage forms for a long time.

Doxylamine succinate also suffers from the disadvantage of having incompatibility with some pharmaceutical excipients such as aspartame, which results in degradation and an increase of impurities in pharmaceutical preparation containing the same.

Doxylamine succinate has an inherently bitter taste, and this constitutes a further disadvantage in particular when formulating certain types of oral preparations. In this sense, it is well known that patients may not complete a necessary course of medicine if they are prescribed an oral presentation which is particularly unpleasant to taste. The bitter taste may be masked by the use of sweetening and/or flavouring agents or lipidic coatings although these are not satisfactory and an unpleasant after-taste may still remain in the mouth. For this reasons, oral formulations of doxylamine succinate are not at the present time entirely satisfactory.

Various methods have been described for masking the bitter taste associated with drug substances, including the use of ion exchange resins.

Ion exchange resins have been also disclosed as means for providing oral formulations with controlled or sustained release properties. In this sense WO 92/11038 (Richarson-Vicks Inc.) discloses oral pharmaceutical preparations which comprise a pharmacologically-active polyamine drug bound to a cation-exchange resin to provide a drug-resin complex having a drug content greater than one equivalent of amine per equivalent of cation-exchange capacity which provides a controllable release of drug under conditions encountered in the gastrointestinal tract (immediate release of the polyamine drug bound in excess of one equivalent and a slower release of the remaining polyamine drug). The cation exchange resin used, Amberlite IRP 69, is a strong acid cation resin, derived from a sulfonated copolymer of styrene and divinylbenzene.

In view of the above stated the problem to be solved by the present invention is to provide a new drugable form of doxylamine which overcomes at least part of the disadvantages above mentioned.

In this respect the inventors have now found that it is possible to form a complex between doxylamine and a synthetic weakly acidic cation exchange resin providing a doxylamine resinate complex. The doxylamine resinate complex

surprisingly presents a decreased hygroscopicity and an improved stability compared to the doxylamine succinate when is combined with some pharmaceutical acceptable excipients which results therefore, in a more stable drugable form of doxylamine. In addition thereto, the resinate complex presents the advantage that it is much easier to administer orally since it is substantially free of the bitter taste associated with doxylamine succinate.

## BRIEF DESCRIPTION OF THE FIGURES

**Figure 1.** Comparative chromatogram of doxylamine succinate
**Figure 2.** Comparative chromatogram of doxylamine polacrilex complex
**Figure 3.** Comparative chromatogram of aspartame
**Figure 4.** Comparative chromatogram of doxylamine succinate:aspartame (1:1)
**Figure 5.** Comparative chromatogram of doxylamine polacrilex complex:aspartame (1:1)

## DETAILED DESCRIPTION OF THE INVENTION

In a first aspect the present invention relates to a doxylamine resinate complex, hereinafter referred to as the resinate complex of the invention, comprising doxylamine bound to a weakly acidic synthetic cation exchange resin.

In principle any weakly acidic synthetic cation exchange resin presenting carboxylic groups can be used to prepare the doxylamine resinate complex as long as it is pharmaceutically acceptable. Preferred weakly acidic synthetic cation exchange resins are polyacrylic copolymers and copolymers of methacrylic acid and divinylbenzene, the latter being more preferred, which can be either synthesized according to well known methods in the art or are commercially available. Examples of resins suitable for use in the resinate complex of the invention are Amberlite IRP64, Amberlite IRP88, Amberlite IRC86, and Amberlite IRC76 (Rohm and Haas). The Amberlite IRP64, hereinafter also referred to as polacrilex, is a preferred resin which presents the following chemical structure:

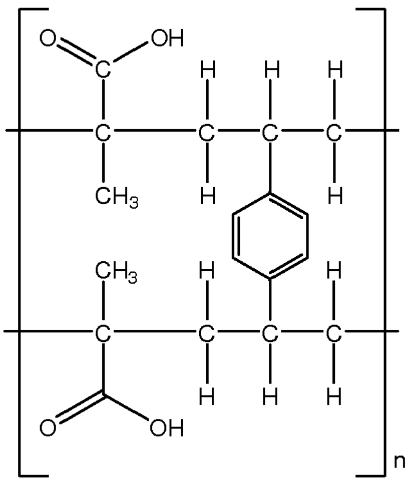

The doxylamine content present in the resinate complex of the invention has been determined and is comprised between 10% by weight and 55% by weight, preferably between 15% by weight and 30% by weight, in respect of the total weight of the doxylamine resinate complex.

The doxylamine complexation efficiency has been determined to be comprised between 80 - 95 %.

The present invention provides in another aspect a process for the preparation of the doxylamine resinate complex of the invention. The process comprises contacting a weakly acidic synthetic cation exchange resin as above disclosed with doxylamine or with a pharmaceutically acceptable salt thereof. The binding may be performed according to known methods for the skilled person, for example as a batch or column process. In a particular embodiment the resinate complex is prepared by a batch process which comprises the steps of contacting the resin previously hydrated in an aqueous buffer solution with doxylamine or a pharmaceutically acceptable salt of doxylamine. The resulting suspension is stirred and maintained until complexation equilibrium is achieved. The resinate complex formed is then filtrated and washed with distilled water to assure elimination of unbound doxylamine and optionally dried, to obtain a fine white powder. The process can be carried out at a range temperature of 25 to 60ºC. Preferably the temperature is comprised between 40-50ºC. The binding step is carried out at a pH value at which the carboxylic groups of the weakly acidic

synthetic cation exchange resin are in ionic form and the doxylamine is protonated. The pH value can thus be easily determined by the skilled person in each case. Typically the pH value is equal or higher than 4. The mobile exchangeable cation in the resin can be a hydrogen ion, ($H^+$) or a cation such as for instance sodium ($Na^+$), potassium ($K^+$), or ammonium ($NH_4^+$).

In a particular embodiment of the process of the invention, the doxylamine is used in the form of the pharmaceutically acceptable salt of doxylamine, more particularly as doxylamine succinate. When doxylamine succinate is used the weight ratio used of doxylamine succinate to the weakly acidic synthetic cation exchange resin ranges from 1:1 to 1:5.

The release of doxylamine from the doxylamine resinate complex added to a release media was determined according to the USP XXIII Paddle Method using a USP Dissolution Tests apparatus 2 (paddles) at 37°C and 50 rpm in 900 mL of 0.1 N HCI during 30 minutes. Dissolution profiles of doxylamine from doxylamine resinate complex showed a fast release, e.g. > 85% of doxylamine was released under the above conditions. The release of doxylamine from the doxylamine resinate complex is similar to the release of doxylamine succinate which means the resinate complex provides an immediate release of doxylamine.

The particle size distribution of doxylamine resinate complex has been determined by Laser Diffraction Analyzer (Malvern Mastersizer 2000) by wet measurement mode. According to a preferred embodiment, particle size of doxylamine resinate complex defined as volume weighted mean D[4,3] is comprised between 20 μm and 300 μm, preferably between 30 μm and 100 μm.

The resinate complex of the invention is used in the preparation of pharmaceutical compositions. It is a more stable drugable form of doxylamine than doxylamine succinate, which is the available physiologically acceptable salt of doxylamine in the market. The resinate complex of the invention is less hygroscopic and more compatible with some common pharmaceutical acceptable carriers, adjuvants or vehicles, generally referred also as excipients. Examples of those excipients are magnesium stearate, stearic acid, povidone K90, lactose and aspartame.

Thus in a further aspect the invention provides a pharmaceutical composition comprising the resinate complex of the invention, hereinafter referred to as the composition of the invention. The composition of the invention further comprises at least one pharmaceutical acceptable excipient.

A pharmaceutical composition according to the present invention may be in any form suitable for the application or administration to humans and/or animals, preferably humans including infants, children and adults, by any route of administration. The composition may be in any dosage form and can vary depending on the route of administration. Examples include, among others, solid forms like powders, tablets, pills, capsules, etc., or liquid forms such as drops, solutions, suspensions, emulsions etc., for oral or topical administration.

The resinate complex of the invention is substantially free of bitter, unpalatable taste, and therefore it is particularly suitable for use in the preparation of pharmaceutical compositions for oral administration a route of administration which is generally preferred for the convenience of the patient. Moreover, the fast dissolution profile of the doxylamine from the resinate complex of the invention as above stated, makes it suitable for its use in oral immediate release pharmaceutical formulations.

The compositions according to the invention can be in form of orally administrable compositions containing one or more physiologically compatible excipients, in solid or liquid form. The compositions may take any convenient form, such as oral pharmaceutical dosage forms like tablets, capsules, lozenges, granules, powders which can be filled in sachets or stick pack, chewable tablets, oral dispersible tablets (ODT), or chewing gums. These compositions may contain conventional excipients such as binding agents, fillers, lubricants, and may also contain further excipients (additives) such as sweeteners, flavourings and preservatives.

Preferred pharmaceutical compositions are chewable tablets, oral dispersible tablets and powders filled in sachets which are convenient dosage forms for most patients having difficulties in swallowing tablets, i.e. children and aged persons.

In another aspect the present invention provides a process for the preparation of the pharmaceutical composition of the invention. Said compositions may be prepared by conventional standard procedures known to those skilled in the art such as those described in the European or US Pharmacopoeias or similar reference texts. Nevertheless as above stated since the new drugable form of doxylamine is less hygroscopic and more stable when is combined with some common pharmaceutical acceptable excipients, pharmaceutical dosage forms comprising the resinate complex of the invention can be advantageously manufactured with no special equipment or atmospheric controls and simplifying the packaging requirements. In addition the resinate complex of the invention is capable of masking the bitter taste associated with doxylamine succinate which is a further advantage in the manufacturing of oral pharmaceutical dosage forms.

Furthermore, it is known that humidity can promote changes in the crystalline form of certain pharmaceutical active ingredients (APIs), such as doxylamine succinate. This effect of a change in the crystalline form can result in a different dissolution profile and/or in less stability, which in turn affect the active ingredient bioavailability. The resinate complex of the invention can avoid this change of crystalline form of the doxylamine succinate.

As above mentioned in a preferred embodiment the pharmaceutical compositions are oral dosage forms, more preferably tablets, like chewable tablets, oral dispersible tablets (ODT) and powders filled in sachets.

The preparation of tablets according to the invention can be achieved according to conventional tabletting techniques, for example dry granulation or wet granulation, and direct compression. The manufacturing of the ODT involves direct compression technology. In the following, percentages are by weight in respect of the total weight of the tablet.

Accordingly to a particular embodiment 10 - 50% of doxylamine resinate complex is blended with at least 30 - 80% of a filler, 3 - 20% of at least a disintegrant, 5 - 50% of at least a binder, 0.1 - 6 % of at least a lubricant, 0.1 - 5% of at least a sweetener and 0.1 - 5 % of at least a flavour. After the blending the blended product is compressed to achieve the tablets.

The fillers, disintegrants, binders, lubricants, sweeteners and flavours suitable for use in these pharmaceutical preparations can be any pharmacologically acceptable ingredients know in the art.

However the filler is preferably selected from sugar alcohols like mannitol, xylitol, isomalt, sorbitol, erythritol, sucrose, fructose, dextrose, threalose, calcium phosphate and is more preferably isomalt or mannitol and also fillers from the family of lactose. The disintegrant is preferably selected from crosspovidone, sodium starch glycolate, croscarmellose sodium, polacrilin potassium, pregelatinized starch, low substituted hydroxypropyl cellulose (L-HPC) and mixtures thereof. The binder is preferably selected from microcrystalline cellulose, corn starch, polyethylene glycol, hydroxypropyl cellulose (HPC), hydroxypropyl methyl cellulose (HPMC), polyvinylpyrrolidone, magnesium aluminium metasilicate, methyl cellulose and mixtures thereof. The lubricant is preferably selected from sodium stearyl fumarate, magnesium stearate, calcium stearate, talc, hydrophilic fumed silica, glyceryl dibehenate, glycerol distearate, behenoyl-polyoxyl glyceride and mixtures thereof. The sweeteners are preferably selected from aspartame, cyclamate, sucrose, sucralose, sodium saccharin and mixtures thereof.

According to a particular embodiment of the invention the pharmaceutical composition is in form of a dosage form for the treatment of occasional insomnia comprising a therapeutically effective amount of doxylamine resinate complex. In the context of the present invention a therapeutically effective amount is to be understood as the amount necessary to achieve a benefice in a treated patient suffering from occasional insomnia. The therapeutically effective amount will depend on several factors such as weight and sex of the sufferer, the severity of the occasional insomnia, etc. However the resinate complex of the invention will typically be administered once or more times a day with typical doses in the range of form 0.1 to 2.5 mg /kg/day.

In another aspect the invention provides the doxylamine resinate complex of the invention for use as a medicament.

In a preferred embodiment said medicament is for the use in the treatment of occasional insomnia.

Inventors have carried out several experiments and have demonstrated the improvements achieved by the invention.

In one experiment (see Example 4) the degree of the hygroscopicity of a resinate complex according to the invention (Sample 2) was compared with the hygroscopicity degree of doxylamine succinate (Sample 1). Both samples 1 and 2 were exposed to humidity conditions for 24 hours and the results from the % increase in mass are shown in the Table 2 of Example 4. It can be seen that in the case of sample 1, sufficient water was absorbed to form a liquid, whereas in sample 2, the same appearance of solid state was observed after the experiment. According to the mentioned scale in Example 4, doxylamine succinate would be deliquescent, whereas doxylamine polacrilex complex would be considered as hygroscopic. Thus it can be concluded that the resinate complex of the invention presents a decreased hygroscopicity compared to the doxylamine succinate.

A further experiment was carried out to evaluate the compatibility of the resinate complex of the invention in comparison with the doxylamine succinate (see Example 5). In particular the compatibility of doxylamine polacrilex complex with aspartame, a potential sweetener excipient to be used in an oral formulation, was compared with the one of doxylamine succinate with the same excipient. Drug - excipient compatibility studies provide information related to real and possible interactions between potential formulation excipients and the active pharmaceutical ingredients (API). The following samples were prepared:

| **Sample** |
|---|
| Doxylamine succinate |
| Doxylamine polacrilex complex |
| Aspartame |
| Doxylamine succinate:Aspartame (1:1) |
| Doxylamine polacrilex complex:Aspartame (1:1) |

And the samples were submitted to different conditions (see the Table 2 from Example 5) wherein Initial refers

to the initial state or appearance of the samples at the beginning of the experiment. Conditions were: 15 days at 40°C by 75% of relative humidity, 15 days at 50ºC, 30 days at 40°C by 75% of relative humidity and 30 days at 50ºC.

The results from the appearance of the samples are summarized in Table 3. It can be seen that the initial appearance of "white powder" of the following samples: doxylamine polacrilex complex, aspartame and binary mixture doxylamine polacrilex complex:aspartame, did not change during the study after any of the conditions. However the initial appearance of "white powder" of the samples: doxylamine succinate and binary mixture doxylamine succinate: aspartame, turned to a whitish wet mass adhering to the vial after 15 days at 40ºC/75%RH, as well as after 30 days at 40ºC/75%RH. Nevertheless the appearance of doxylamine succinate did not change after 15 days or 30 days at 50ºC, and changed slightly for binary mixture doxylamine succinate:aspartame.

The samples were also submitted to the ultra performance liquid chromatography (UPLC) analysis at the initial state and after their submission to the different conditions. The results of the comparatives chromatograms for each sample are shown in Tables 4, 5, 6, 7 and 8 and the corresponding comparative chromatograms are shown in Figures 1 to 5.

Concerning the ultra performance liquid chromatography (UPLC) analysis, related substance 1 is a substance which is already observed in the initial product and its amount did not increase during the study in any of the samples studied under any of the conditions (except for the individual aspartame, which did not show this impurity).

Individual doxylamine succinate (Table 4) showed some degradation after 30 days at 40ºC/75%RH while the other individual samples, doxylamine polacrilex complex and aspartame, did not show any degradation during the study. The improved stability of the resinate complex of the invention is readily recognized.

As far as the binary mixtures doxylamine succinate:aspartame and doxylamine polacrilex complex:aspartame are concerned, the following was observed.

Doxylamine succinate:aspartame (1:1) (Table 7) showed some degradation of the doxylamine succinate component (see the presence of related substances 2, 3, 4 and 5) after 15 and 30 days at 40ºC/75%RH. It can be also observed after 15 and 30 days at 40ºC/75%RH a conversion of aspartame (appearance of substances 6 to 9) probably due to equilibrium between the zwitterionic form and the partially ionized forms. At the condition studied of 15 days or 30 days at 50°C, doxylamine succinate:aspartame (1:1) did not show major changes during the study.

On the contrary, for the binary mixture doxylamine polacrilex complex:aspartame (1:1) (Table 8) none significant changes were observed at all during the study after submitting this sample to the exposed conditions.

From the observed results it can be concluded that a clear interaction was observed in the binary mixture of Doxylamine succinate:Aspartame (1:1) when the sample was exposed to 40ºC/75%RH, whereas no interaction between excipient and the resinate complex of the invention was detected in particular in the binary mixture of Doxylamine polacrilex complex:Aspartame (1:1). Therefore the resinate complex of the invention shows increased compatibility with some excipients, such as aspartame.

Therefore a further aspect the invention provides the use of doxylamine resinate complex to increase the excipient compatibility of doxylamine. In a particular embodiment the excipient is selected from magnesium stearate, stearic acid, povidone K90, lactose and aspartame. In a preferred embodiment, the excipient is aspartame.

In still a further aspect the invention provides the use of doxylamine resinate complex to decrease hygroscopicity of doxylamine.

The foregoing is illustrative of the present invention. This invention however is not limited to the following precise embodiments described herein, but encompasses all equivalent modifications within the scope of the claims which follow.

## EXAMPLES

***<u>Example 1:</u> Doxylamine - Polacrilex complex preparation.***

8,72 Kg of Amberlite IRP64 were hydrated in 216 L of pH 8 Phosphate Buffer previously heated to 45 - 50 °C for 45 minutes. 3,49 Kg of doxylamine succinate were added to the suspension and stirring was maintained during 5 hours at 45 - 50 °C to achieve the complexation equilibrium (drug:resin ratio is 1:2,5).

The suspension was filtered through a centrifuge. The cake was washed with 10 L of distilled water and dried in a rotary drier at 60 °C.

The complex Amberlite IRP64 - doxylamine is obtained as a fine white powder containing from 18.7 % of doxylamine free base.

The doxylamine complexation efficiency obtained is 90.3 %.

***<u>Example 2:</u> Doxylamine - Polacrilex Oral Dispersible Tablet.***

The oral disintegrating tablets containing doxylamine - Amberlite IRP64 complex were prepared by direct compression technology. The doxylamine - Amberlite IRP64 complex was blended with mannitol, crosspovidone (or

sodium starch glycolate), microcrystalline cellulose, sodium stearyl fumarate, aspartame and flavour.

The blended product was compressed to achieve tablets with the following composition.

| | % (w/w) |
|---|---|
| Doxylamine -Polacrilex complex | 25.0 |
| Mannitol | 50.0 |
| Microcrystalline Cellulose | 14.5 |
| Crosspovidone | 7.0 |
| Sodium stearyl fumarate | 1.5 |
| Aspartame | 1.0 |
| Flavour | 1.0 |

***Example 3:* *Doxylamine - Polacrilex complex Chewable Tablet.***

The chewable tablets containing Doxylamine - Amberlite IRP64 complex were prepared by direct compression technology. The doxylamine - Amberlite IRP64 complex was blended with soluble isomalt, polyethylene glycol, sodium stearyl fumarate, aspartame and flavours. The blended product is compressed to achieve tablets

The blended product was compressed to achieve tablets with the following composition:

| | %(w/w) |
|---|---|
| Doxylamine - Polacrilex complex | 25.0 |
| Isomalt | 63.5 |
| Polyethylene Glycol | 8.0 |
| Sodium Stearyl Fumarate | 1.5 |
| Aspartame | 1.0 |
| Flavour | 1.0 |

**Example 4: Determination of the degree of hygroscopicity of doxylamine succinate and doxylamine polacrilex complex according to the invention:**

The objective of the experiment was to measure the degree of hygroscopicity of the doxylamine polacrilex complex and comparing it with the one of the doxylamine succinate. The percentage increase of mass was calculated in order to evaluate the adsorption of water in the samples.

According to "5.11 Characters section in monographs" in European Pharmacopeia 6.0, the degree of hygroscopicity of a substance is defined based on the percentage increase of mass of the substance after 24 hours of exposure at 80 $\pm$ 2 per cent relative humidity and 25 $\pm$ 1ºC. The result is interpreted as follows:

Table 1. Classification of substances concerning hygroscopicity

| **Percentage increase in mass** | **Degree of hygroscopicity** |
|---|---|
| $\Delta$ < 0,2% | not hygroscopic |
| 0,2%< $\Delta$ < 2% | slightly hygroscopic |
| 2%< $\Delta$ < 15% | Hygroscopic |
| 15%< $\Delta$ | very hygroscopic |
| Dissolved in water | Deliquescent |

**Materials and Methods:**

Dynamic Vapour Sorption (DVS) analysis is a technique specifically designed to provide information related to material response to changes in relative humidity and TºC. A Q5000 SA DVS equipment from TA Instruments was used for running the experiment.

A program was configured to run the analysis at 25ºC and 80%RH for 24h. A previous program at 60ºC was

run until constant weight of the sample in order to begin the analysis with the dried sample.

The samples analysed were:

Sample 1: doxylamine succinate powder (drug)
Sample 2: doxylamine polacrilex complex powder (drug:resin complex)

**Results:**

The variation of the weight is controlled and the following percentage is calculated:

$$\Delta \%weight = (P_n - P_1)/P_1 \times 100$$

wherein: $P_1$ is the initial weight, $P_n$ is the weight measured for the sample.
The results obtained are summarized in the following table:

Table 2: Results % increase in mass

| **Sample** | **Δ %weight 24 h** |
|---|---|
| Sample 1 | 29-30% |
| Sample 2 | 10.0-15.0% |

In the case of sample 1, sufficient water was absorbed to form a liquid. In sample 2, same appearance of solid state was observed after the experiment.

According to the above mentioned scale, doxylamine succinate would be deliquescent, whereas doxylamine polacrilex complex would be considered as hygroscopic.

**Example 5: Drug - excipient compatibility study**

The objective of the study was to measure the compatibility of doxylamine polacrilex complex with aspartame, a potential sweetener excipient to be used in an oral formulation, and comparing it with the one of doxylamine succinate with the same excipient.

Drug - excipient compatibility studies provide information related to real and possible interactions between potential formulation excipients and the API.

**Materials and Methods:**

Samples

Sufficient amount of individual substances and binary mixtures were accurately weighted in appropriate vials (5 vials for each sample). Samples prepared are listed in the following table:

Table 1. Samples prepared for the drug:excipient compatibility study

| **Sample** | **Amount per vial** |
|---|---|
| Doxylamine succinate | 50 mg |
| Doxylamine polacrilex complex | 180 mg* |
| Aspartame | 50 mg |
| Doxylamine succinate:Aspartame (1:1) | 50 + 50 mg |

(continued)

| Sample | Amount per vial |
|---|---|
| Doxylamine polacrilex complex:Aspartame (1:1) | 180* + 180 mg |
| * The amount of active substance present in 180 mg of Doxylamine polacrilex complex is approximately equivalent to the amount of active substance present in 50 mg of Doxylamine succinate. | |

Storage and Analysis

One of the vials was used for initial analysis, 2 closed vials were placed in an oven at 50ºC and the other 2 (open vials, to maximize the effect of the humidity) were placed in climatic chamber at 40ºC/75%RH. Appearance, active substance assay and related substances (UPLC) were tested during the study. Storage and frequency of analyses are summarized in the following table:

Table 2. Storage and frequency of analysis

| Days / Storage Conditions | Initial | 15 days | 30 days |
|---|---|---|---|
| Oven 50ºC | ☒ | ☒ | ☒ |
| 40ºC/75%RH | | ☒ | ☒ |

**Results**

Appearance

Initially, all samples studied were observed as white powder. Appearance of the samples during the study is summarized in the following table:

Table 3. Results for Appearance

| Sample | Initial | 15 days 40ºC/ 75%R H | 15 days 50ºC | 30 days 40ºC/ 75%R H | 30 days 50ºC |
|---|---|---|---|---|---|
| **Doxylamine succinate** | White powder | whitish mass adhered to the vial | White powder | colourless mass adhered to the vial | White powder |
| **Doxylamine polacrilex complex** | White powder | White powder | White powder | White powder | White powder |
| **Aspartame** | White powder | White powder | White powder | White powder | White powder |
| **Doxylamine succinate: Aspartame (1: 1)** | White powder | whitish mass and some white powder adhered to the vial | White powder slightly adhered | whitish mass and some white powder adhered to the vial | White powder slightly adhered |
| **Doxylamine polacrilex complex: Aspartame (1: 1)** | White powder | White powder | White powder | White powder | White powder |

Assay and related products (UPLC)

Peaks that appeared in the chromatogram (rejecting peaks that matched with the blank solution) were quantified by % area. Results obtained are summarized in the following tables:

Table 4. Comparative chromatogram of Doxylamine succinate

| % Area value | INITIAL | 15 days 40°C/75%RH | 15 days 50°C | 30 days 40°C/75%RH | 30 days 50°C |
|---|---|---|---|---|---|
| **300241** | 99.70 | 99.67 | 99.72 | 99.53 | 99.67 |
| **Ref. subst. 1** | 0.30 | 0.33 | 0.28 | 0.30 | 0.30 |
| **Ref. subst. 2** | - | - | - | 0.02 | - |
| **Ref. subst. 3** | - | - | - | 0.09 | 0.02 |
| **Ref. subst. 4** | - | - | - | 0.06 | 0.01 |

Table 5. Comparative chromatogram of doxylamine polacrilex complex

| % Area value | INITIAL | 15 days 40°C/75%RH | 15 days 50°C | 30 days 40°C/75%RH | 30 days 50°C |
|---|---|---|---|---|---|
| **30241** | 99.82 | 99.82 | 99.82 | 99.82 | 99.84 |
| **Ref. subst. 1** | 0.18 | 0.18 | 0.18 | 0.18 | 0.16 |

Table 6. Comparative chromatogram of aspartame

| % Area value | INITIAL | 15 days 40°C/75%RH | 15 days 50°C | 30 days 40°C/75%RH | 30 days 50°C |
|---|---|---|---|---|---|
| **Aspartame** | 95.26 | 100.00 | 100.00 | 100.00 | 98.52 |
| **Ref. subst. 6** | 4.74 | - | - | - | 1.48 |

Chromatograms of the samples analysed at different time points of the study are shown in the following figures 1 to 5.

**Claims**

1. A doxylamine resinate complex comprising doxylamine bound to a weakly acidic synthetic cation exchange resin.

2. A doxylamine resinate complex according to claim 1 wherein said weakly acidic synthetic cation exchange resin is a copolymer of methacrylic acid and divinylbenzene.

3. A doxylamine resinate complex according to claim 1 wherein the doxylamine amount is comprised between 10% by weight and 55% by weight, preferably between 15% by weight and 30% by weight, in respect of the total weight of the doxylamine resinate complex.

4. A process for the preparation of the doxylamine resinate complex according to claim 1 comprising contacting a weakly acidic synthetic cation exchange resin with doxylamine or a pharmaceutically acceptable salt thereof.

5. A process according to claim 4 wherein the doxylamine used is in the form of the pharmaceutically acceptable salt of doxylamine, doxylamine succinate.

6. A process according to claim 5 wherein weight ratio used of doxylamine succinate to the weakly acidic synthetic cation exchange resin ranges from 1:1 to 1:5.

7. A doxylamine resinate complex according to anyone of claims 1 to 3 for use as a medicament.

8. A doxylamine resinate complex according to claim 7 for use in the treatment of occasional insomnia.

9. A pharmaceutical composition which comprises doxylamine resinate complex according to claim 1 and at least a pharmaceutically acceptable excipient.

10. A pharmaceutical composition according to claim 9 for oral administration.

11. A pharmaceutical composition according to claim 10, wherein said pharmaceutical composition provides an immediate release of doxylamine.

12. A pharmaceutical composition according to claim 10, wherein said pharmaceutical composition is an oral dispersible tablet, a chewable tablet or powder in sachets.

13. A pharmaceutical composition according to any one of claims 9 to 11, for the treatment of occasional insomnia which comprises an effective amount of doxylamine resinate complex.

14. Use of doxylamine resinate complex according to any one of claims 1 to 3 to increase the excipient compatibility of doxylamine.

15. Use of doxylamine resinate complex according to any one of claims 1 to 3, to decrease hygroscopicity of doxylamine.

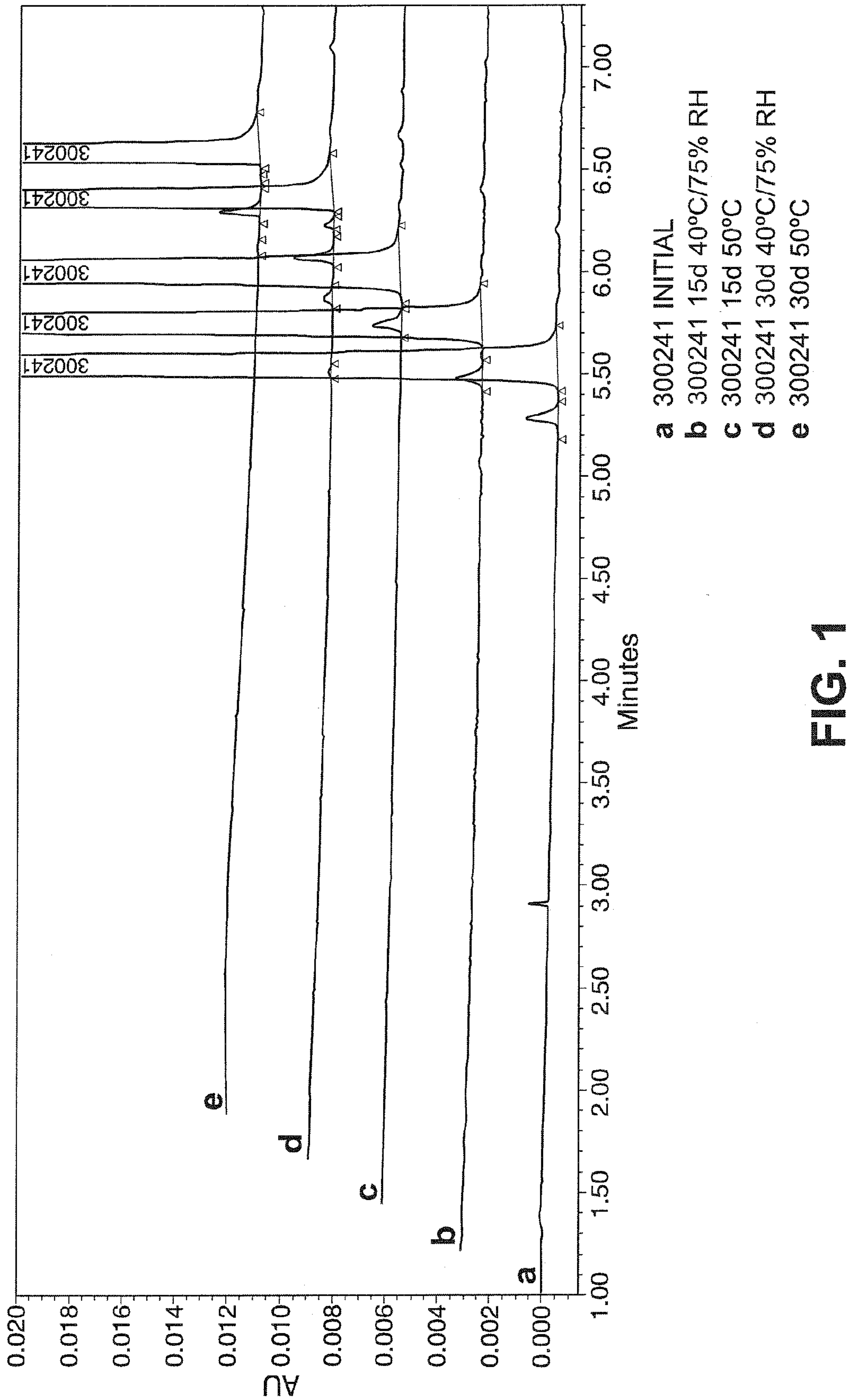
a 300241 INITIAL
b 300241 15d 40°C/75% RH
c 300241 15d 50°C
d 300241 30d 40°C/75% RH
e 300241 30d 50°C
Minutes
AU
300241

FIG. 1

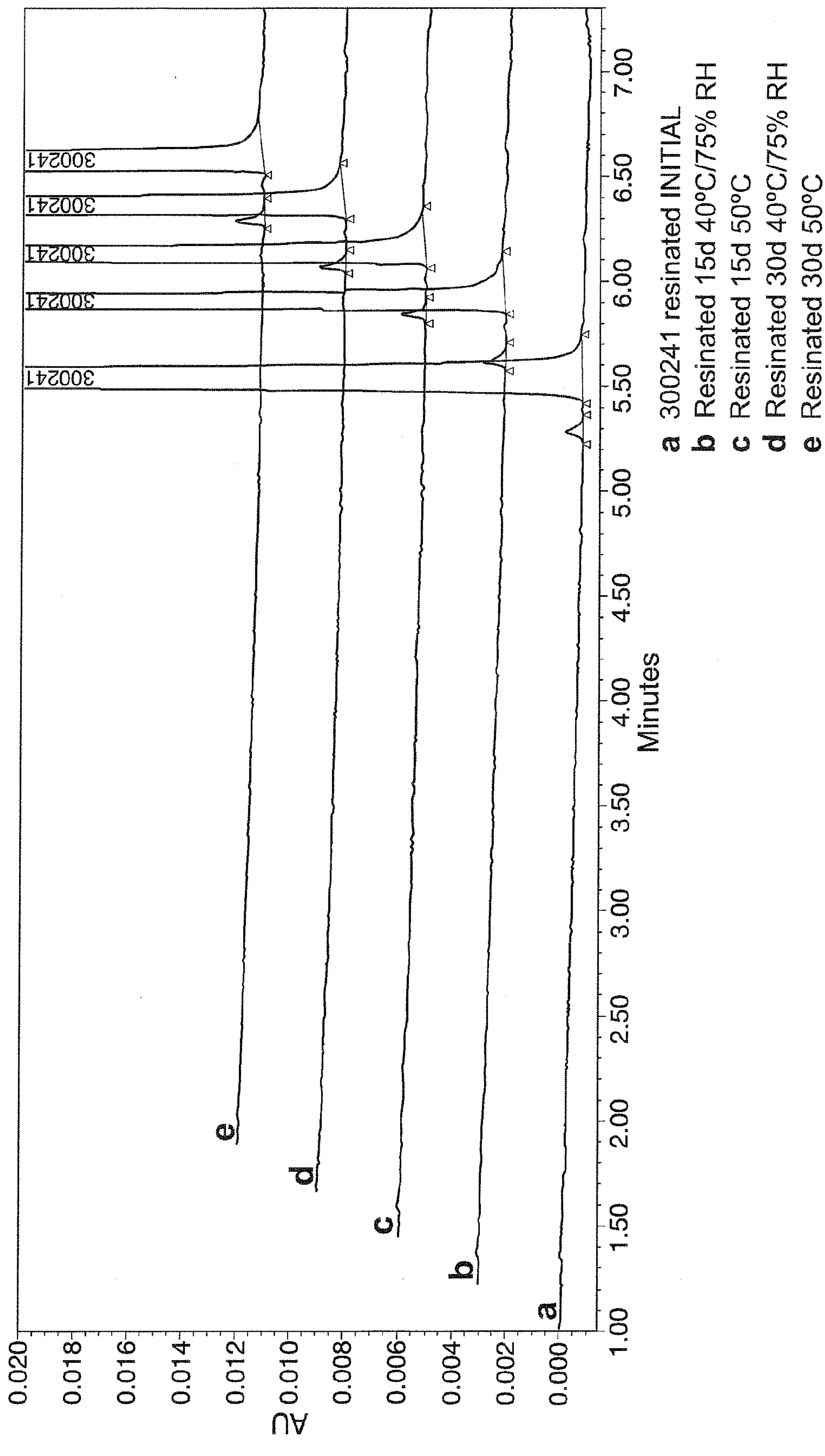
AU
0.020
0.018
0.016
0.014
0.012
0.010
0.008
0.006
0.004
0.002
0.000
Minutes
1.00
1.50
2.00
2.50
3.00
3.50
4.00
4.50
5.00
5.50
6.00
6.50
7.00
300241
a
b
c
d
e
a 300241 resinated INITIAL
b Resinated 15d 40°C/75% RH
c Resinated 15d 50°C
d Resinated 30d 40°C/75% RH
e Resinated 30d 50°C

FIG. 2

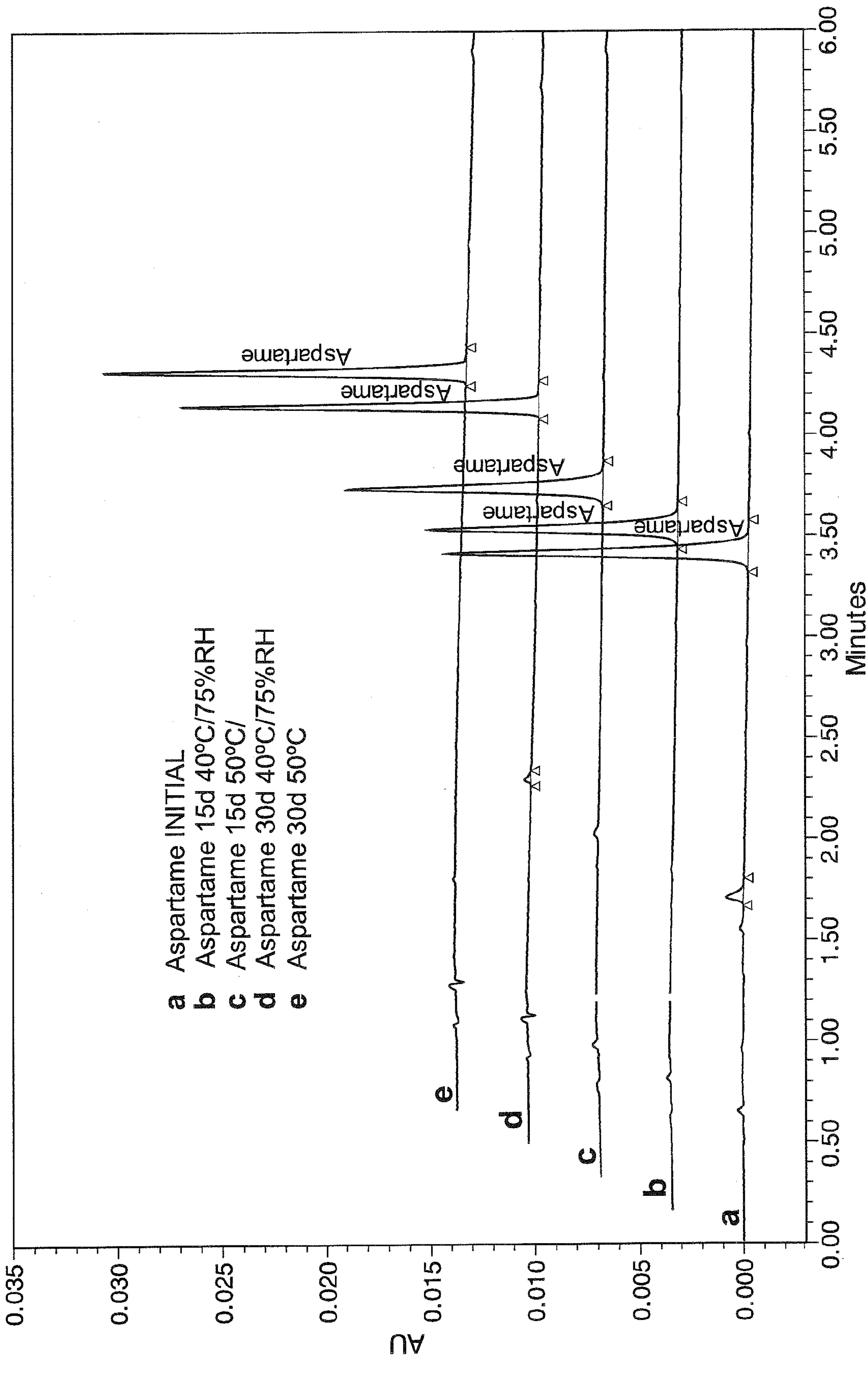
a Aspartame INITIAL
b Aspartame 15d 40°C/75%RH
c Aspartame 15d 50°C/
d Aspartame 30d 40°C/75%RH
e Aspartame 30d 50°C
Aspartame
AU
Minutes
0.000
0.005
0.010
0.015
0.020
0.025
0.030
0.035
0.00
0.50
1.00
1.50
2.00
2.50
3.00
3.50
4.00
4.50
5.00
5.50
6.00

FIG. 3

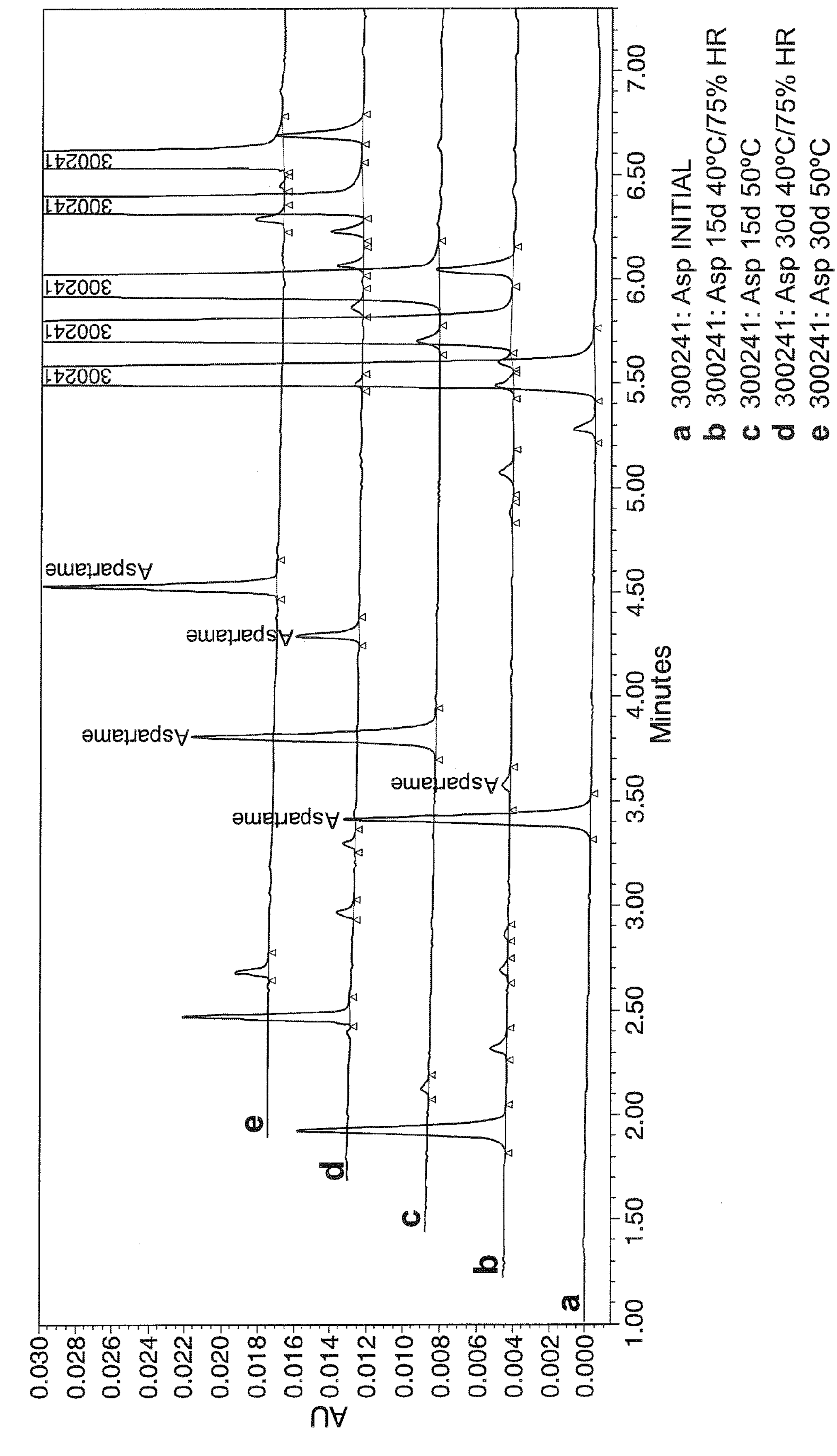
a 300241: Asp INITIAL
b 300241: Asp 15d 40°C/75% HR
c 300241: Asp 15d 50°C
d 300241: Asp 30d 40°C/75% HR
e 300241: Asp 30d 50°C
Aspartame
300241
AU
Minutes
0.000
0.002
0.004
0.006
0.008
0.010
0.012
0.014
0.016
0.018
0.020
0.022
0.024
0.026
0.028
0.030
1.00
1.50
2.00
2.50
3.00
3.50
4.00
4.50
5.00
5.50
6.00
6.50
7.00
a
b
c
d
e

FIG. 4

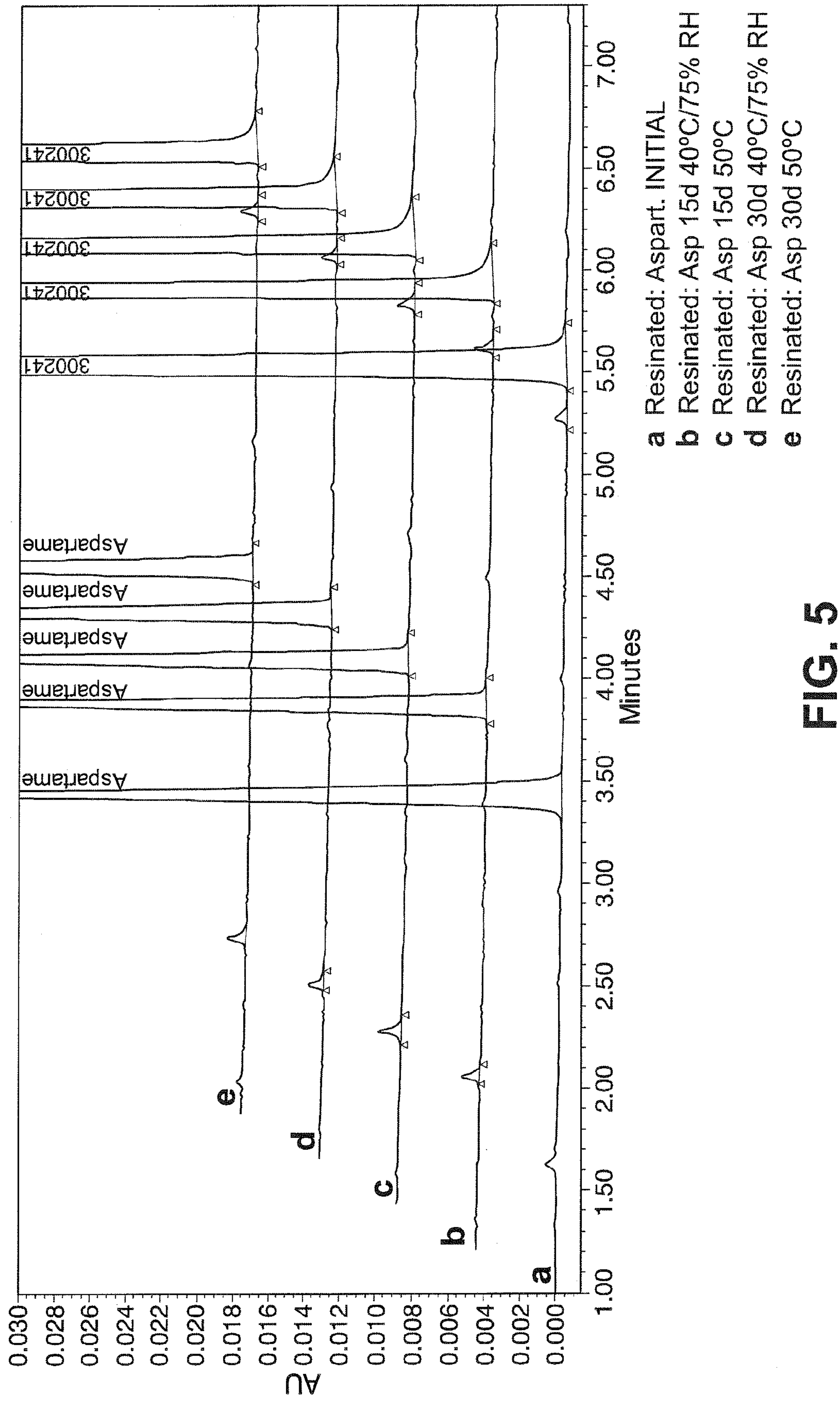
AU
0.030
0.028
0.026
0.024
0.022
0.020
0.018
0.016
0.014
0.012
0.010
0.008
0.006
0.004
0.002
0.000
Minutes
1.00
1.50
2.00
2.50
3.00
3.50
4.00
4.50
5.00
5.50
6.00
6.50
7.00
Aspartame
300241
a
b
c
d
e
a Resinated: Aspart. INITIAL
b Resinated: Asp 15d 40°C/75% RH
c Resinated: Asp 15d 50°C
d Resinated: Asp 30d 40°C/75% RH
e Resinated: Asp 30d 50°C

FIG. 5

Europäisches Patentamt
European Patent Office
Office européen des brevets

# EUROPEAN SEARCH REPORT

Application Number
EP 11 38 2072

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br><br><br><br><br><br><br><br><br><br><br>Y | PUTTEWAR T Y ET AL: "Formulation and evaluation of orodispersible tablet of taste masked doxylamine succinate using ion exchange resin", MAGALLAT GAMI'AT AL-MALIK SA'UD. AL-'ULUM - JOURNAL OF KING SAUDUNIVERSITY. SCIENCE, GAMI'AT AL-MALIK SA'UD, RIYAD, SA, vol. 22, no. 4, 1 October 2010 (2010-10-01), pages 229-240, XP027275272, ISSN: 1018-3647 [retrieved on 2010-05-15]<br>* page 231, right-hand column, paragraph 2.2.2 *<br>* page 231, right-hand column, paragraph 2.2.2.3 *<br>* page 232, left-hand column, paragraph 2.2.2.6 *<br>* page 230, left-hand column, paragraph 3 *<br>* page 234; table 6 *<br>* page 237, left-hand column, paragraph 2.2.6.7; table 10 *<br>----- | 1-12<br><br><br><br><br><br><br><br><br><br><br><br>13-15 | INV.<br>A61K31/135<br>A61K31/4402<br>A61K9/00<br>A61K9/08<br>A61K9/10<br>A61K9/20<br>A61K9/48<br>A61K47/48<br><br>ADD.<br>A61P25/20 |
| Y | GOULD ET AL: "Salt selection for basic drugs", INTERNATIONAL JOURNAL OF PHARMACEUTICS, ELSEVIER BV, NL, vol. 33, no. 1-3, 1 November 1986 (1986-11-01), pages 201-217, XP025813036, ISSN: 0378-5173, DOI: DOI:10.1016/0378-5173(86)90055-4 [retrieved on 1986-11-01]<br>* page 211; table 5 *<br>* page 213, left-hand column, paragraph 2 *<br>-----<br>-/-- | 1-15 | TECHNICAL FIELDS SEARCHED (IPC)<br>A61K<br>A61P |

The present search report has been drawn up for all claims

| Place of search | Date of completion of the search | Examiner |
|---|---|---|
| Munich | 9 August 2011 | Tullberg, Erik |

3

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 11 38 2072

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | DATABASE WPI<br>Week 200580<br>Thomson Scientific, London, GB;<br>AN 2005-783232<br>XP002653988,<br>& JP 2005 325070 A (SS PHARM CO)<br>24 November 2005 (2005-11-24)<br>* abstract *<br>----- | 1-15 | |
| A,D | WO 92/11038 A1 (RICHARDSON VICKS INC [US])<br>9 July 1992 (1992-07-09)<br>* example 1 *<br>----- | 1-15 | |
| A | VAN TONDER E C ET AL: "Polymorphism of doxylamine succinate and X-ray structural study of Form I and of doxylamine succinate 0.5 succinic acid",<br>INTERNATIONAL JOURNAL OF PHARMACEUTICS, ELSEVIER BV, NL,<br>vol. 63, no. 1,<br>31 August 1990 (1990-08-31), pages 35-42,<br>XP025544051,<br>ISSN: 0378-5173, DOI:<br>DOI:10.1016/0378-5173(90)90098-O<br>[retrieved on 1990-08-31]<br>* the whole document *<br>----- | 1-15 | TECHNICAL FIELDS SEARCHED (IPC) |
| A | VAN TONDER E C ET AL: "COMPATIBILITY STUDY BETWEEN DOXYLAMINE SUCCINATE WITH OTHER DRUGS AND EXCIPIENTS USING DIFFERENTIAL SCANNING CALORIMETRY",<br>DRUG DEVELOPMENT AND INDUSTRIAL PHARMACY, NEW YORK, NY, US,<br>vol. 16, no. 14,<br>1 January 1990 (1990-01-01), pages 2125-2133, XP009150786,<br>ISSN: 0363-9045<br>* the whole document *<br>----- | 1-15 | |

The present search report has been drawn up for all claims

3

| Place of search | Date of completion of the search | Examiner |
|---|---|---|
| Munich | 9 August 2011 | Tullberg, Erik |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO. EP 11 38 2072

This annex lists the patent family members relating to the patent documents cited in the above-mentioned European search report.
The members are as contained in the European Patent Office EDP file on
The European Patent Office is in no way liable for these particulars which are merely given for the purpose of information.

09-08-2011

| Patent document cited in search report | Publication date | Patent family member(s) | Publication date |
|---|---|---|---|
| JP 2005325070 A | 24-11-2005 | NONE | |
| WO 9211038 A1 | 09-07-1992 | AU 663695 B2 | 19-10-1995 |
| | | AU 9168791 A | 22-07-1992 |
| | | BR 9107173 A | 16-11-1993 |
| | | CA 2098200 A1 | 21-06-1992 |
| | | CN 1063221 A | 05-08-1992 |
| | | CZ 9301197 A3 | 19-01-1994 |
| | | EP 0563294 A1 | 06-10-1993 |
| | | FI 932842 A | 18-06-1993 |
| | | HU 74642 A2 | 28-01-1997 |
| | | IE 914513 A1 | 01-07-1992 |
| | | JP 6504059 T | 12-05-1994 |
| | | NZ 241097 A | 25-11-1994 |
| | | PT 99912 A | 29-01-1993 |
| | | SK 59493 A3 | 10-11-1993 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9211038 A